# EUROPEAN PATENT SPECIFICATION

(11) **EP 4 395 881 B1**
(45) Date of publication and mention of the grant of the patent: **11.03.2026**
(21) Application number: 22769386.8
(22) Date of filing: 25.08.2022
(51) Int. Cl.: A61N 1/36, A61N 1/05

(54) **DETECTION OF NEURAL POTENTIAL EVOKED IN RESPONSE TO ELECTRICAL STIMULATION**
NACHWEIS EINES ALS REAKTION AUF ELEKTRISCHE STIMULATION EVOZIERTEN NEURONALEN POTENZIALS
DÉTECTION D'UN POTENTIEL NEURONAL ÉVOQUÉ EN RÉPONSE À UNE STIMULATION ÉLECTRIQUE

(30) Priority: 02.09.2021 US 202163240291 P; 15.08.2022 US 202217819798
(43) Date of publication of application: 10.07.2024
(73) Proprietor: Medtronic, Inc., Minneapolis, Minnesota 55432 (US)
(72) Inventor: LITVAK, Leonid M., Minneapolis, Minnesota 55432 (US); STANSLASKI, Scott R., Minneapolis, Minnesota 55432 (US); PETERSON, Erik J., Minneapolis, Minnesota 55432 (US)
(74) Representative: Zimmermann & Partner Patentanwälte mbB
(86) International application number: PCT/IB2022/057968
(87) International publication number: WO 2023/031740

(56) References cited:
- WO-A1-02/36003
- WO-A1-2019/210371
- US-A1- 2012 271 189
- US-A1- 2021 121 698

## Description

### TECHNICAL FIELD

This disclosure generally relates to electrical stimulation and recording.

### BACKGROUND

Medical devices may be external or implanted, and may be used to deliver electrical stimulation therapy to various tissue sites of a patient to treat a variety of symptoms or conditions such as chronic pain, tremor, Parkinson's disease, other movement disorders, epilepsy, urinary or fecal incontinence, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Hence, electrical stimulation may be used in different therapeutic applications, such as deep brain stimulation (DBS), spinal cord stimulation (SCS), sacral nerve stimulation (SNS), tibial nerve stimulation (TNS), gastric stimulation, or peripheral nerve field stimulation (PNFS).

A clinician may select values for a number of programmable parameters in order to define the electrical stimulation therapy to be delivered by the implantable stimulator to a patient. For example, the clinician may select electrodes for delivery of the stimulation, a polarity of each selected electrode, a voltage or current amplitude, a pulse width, and a pulse frequency as stimulation parameters. A set of parameters, such as a set including electrode combination, electrode polarity, voltage or current amplitude, pulse width and pulse rate, may be referred to as a program in the sense that they define the electrical stimulation therapy to be delivered to the patient. Document US 2012/271189 A1 relates to a system configured to sense a signal and to determine a variance of the signal amplitude.

### SUMMARY

The present invention is defined by the appended claims and relates to a system and a computer-readable storage medium. The method described herein is useful to understand the invention and relates to the computer-readable storage medium. In one example, a method includes delivering, by an implantable stimulation device, a plurality of doses of electrical stimulation to a patient; sensing, by the implantable stimulation device and for each respective dose of the plurality of doses, a respective electrical signal of a plurality of electrical signals; and determining, based on a variation of the plurality of electrical signals, whether the plurality of doses of electrical stimulation evoked neural potentials in the patient.

In another example, a system includes a memory; and processing circuitry configured to: cause an implantable stimulation device to deliver a plurality of doses of electrical stimulation to a patient; receive, for each respective dose of the plurality of doses, a respective electrical signal of a plurality of electrical signals; and determine, based on a variation of the plurality of electrical signals, whether the plurality of doses of electrical stimulation evoked neural potentials in the patient.

In another example, a computer-readable storage medium includes instructions that, when executed, cause processing circuitry to: cause an implantable stimulation device to deliver a plurality of doses of electrical stimulation to a patient; sense, via the implantable stimulation device and for each respective dose of the plurality of doses, a respective electrical signal of a plurality of electrical signals; and determine, based on a variation of the plurality of electrical signals, whether the plurality of doses of electrical stimulation evoked neural potentials in the patient.

The summary is intended to provide an overview of the subject matter described in this disclosure. It is not intended to provide an exclusive or exhaustive explanation of the systems, device, and methods described in detail within the accompanying drawings and description below. Further details of one or more examples of this disclosure are set forth in the accompanying drawings and in the description below. Other features, objects, and advantages will be apparent from the description and drawings, and from the claims.

### BRIEF DESCRIPTION OF DRAWINGS

FIG. 1 is a conceptual diagram illustrating an example system that includes an implantable medical device (IMD) configured to deliver deep brain stimulation (DBS) to a patient according to an example of the techniques of the disclosure.
FIG. 2 is a block diagram of the example IMD of FIG. 1 for delivering DBS therapy according to an example of the techniques of the disclosure.
FIG. 3 is a block diagram of the external programmer of FIG. 1 for controlling delivery of DBS therapy according to an example of the techniques of the disclosure.
FIG. 4 is a flowchart illustrating an example technique for sensing evoked neural potentials, in accordance with one or more techniques of this disclosure.
FIG. 5 is a graph illustrating example sensed electrical signals and their variances, in accordance with one or more techniques of this disclosure.

### DETAILED DESCRIPTION

In general, the disclosure describes devices, systems, and techniques for neural sensing. Sensed electrical signals can serve as inputs for generation of control signals for electrical stimulation therapies. Neural potentials evoked in response to electrical stimulation (such as evoked compound action potentials (ECAPs), evoked resonant neural activity (ERNA) or other evoked physiological signals) describe the network response of the central nervous system (CNS) to electrical stimulation. However, these potentials can take a long time to evolve (e.g., 40 milliseconds) whereas the typical stimulation paradigms have relatively high stimulation rates (e.g., 150 Hz pulse rate for deep brain stimulation (DBS)) and hence may not be amenable for recording relatively long responses. In addition, neural responses can be masked by large stimulation artifact which makes it difficult to capture their features or even detect their presence, especially in cases when the bandwidth (number of samples per seconds of data captured) is limited.

In accordance with one or more techniques of this disclosure, as opposed to directly using measurements (e.g., voltage measurements) of evoked neural potentials in response to electrical stimulation, an implantable medical device (IMD) may utilize variances of the measurements. The parameters of the stimulation delivered to the patient may be nearly identical every time, e.g., with each stimulation pulse or burst. However, as they are biological, the evoked neural potentials may vary slightly every time or some of the time, e.g., with each stimulation pulse. This variation may become apparent when examining measurements captured after several stimulation deliveries. The IMD may examine variability across time to distinguish second-order potentials, obtained from the sensed potentials, from artifact and immediate synchronized response. Increases in variation in the response may imply second order responses to neural stimulation. The use of variation measure becomes particularly useful when under-sampling the signal where neural signal features are less recognizable. Examples of variation include, but are not limited to, standard deviation and variance. The IMD may perform closed-loop stimulation based on results of the sensing, e.g., by formulating, in response to sensed second-order potentials, control signals to adjust one or more parameter values of the stimulation, such as pulse amplitude, pulse width, or pulse rate. The techniques of this disclosure may be applicable to different therapeutic applications, such as deep brain stimulation (DBS), spinal cord stimulation (SCS), sacral nerve stimulation (SNS), tibial nerve stimulation (TNS), gastric stimulation, or peripheral nerve field stimulation (PNFS).

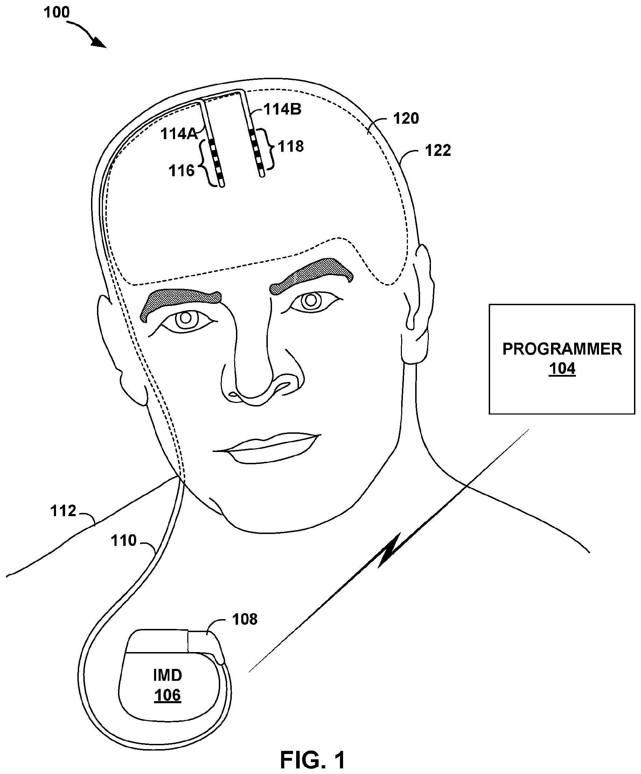

FIG. 1 is a conceptual diagram illustrating an example system 100 that includes an implantable medical device (IMD) 106 configured to deliver DBS to patient 122 according to an example of the techniques of the disclosure. As shown in the example of FIG. 1, example system 100 includes medical device programmer 104, implantable medical device (IMD) 106, lead extension 110, and leads 114A and 114B with respective sets of electrodes 116, 118. In the example shown in FIG. 1, electrodes 116, 118 of leads 114A, 114B are positioned to deliver electrical stimulation to a tissue site within brain 120, such as a deep brain site under the dura mater of brain 120 of patient 112. In some examples, delivery of stimulation to one or more regions of brain 120, such as the subthalamic nucleus, globus pallidus or thalamus, may be an effective treatment to manage movement disorders, such as Parkinson's disease, epilepsy, etc. Some or all of electrodes 116, 118 also may be positioned to sense neurological brain signals within brain 120 of patient 112. In some examples, some of electrodes 116, 118 may be configured to sense neurological brain signals and others of electrodes 116, 118 may be configured to deliver adaptive electrical stimulation to brain 120. In other examples, all of electrodes 116, 118 are configured to both sense neurological brain signals and deliver adaptive electrical stimulation to brain 120.

IMD 106 includes a therapy module (e.g., which may include processing circuitry, signal generation circuitry or other electrical circuitry configured to perform the functions attributed to IMD 106) that includes a stimulation generator configured to generate and deliver electrical stimulation therapy to patient 112 via a subset of electrodes 116, 118 of leads 114A and 114B, respectively. The subset of electrodes 116, 118 that are used to deliver electrical stimulation to patient 112, and, in some cases, the polarity of the subset of electrodes 116, 118, may be referred to as a stimulation electrode combination. As described in further detail below, the stimulation electrode combination can be selected for a particular patient 112 and target tissue site (e.g., selected based on the patient condition). The group of electrodes 116, 118 includes at least one electrode and can include a plurality of electrodes. In some examples, the plurality of electrodes 116 and/or 118 may have a complex electrode geometry such that two or more electrodes of the lead are located at different positions around the perimeter of the respective lead (e.g., different positions around a longitudinal axis of the lead).

In some examples, the neurological signals (e.g., an example type of electrical signals) sensed within brain 120 may reflect changes in electrical current produced by the sum of electrical potential differences across brain tissue. Examples of neurological brain signals include, but are not limited to, electrical signals generated from local field potentials (LFP) sensed within one or more regions of brain 120, such as an electroencephalogram (EEG) signal, or an electrocorticogram (ECoG) signal. Local field potentials, however, may include a broader genus of electrical signals within brain 120 of patient 112.

In some examples, the neurological brain signals that are used to select a stimulation electrode combination may be sensed within the same region of brain 120 as the target tissue site for the electrical stimulation. As previously indicated, these tissue sites may include tissue sites within anatomical structures such as the thalamus, subthalamic nucleus or globus pallidus of brain 120, as well as other target tissue sites. The specific target tissue sites and/or regions within brain 120 may be selected based on the patient condition. Thus, due to these differences in target locations, in some examples, the electrodes used for delivering electrical stimulation may be different than the electrodes used for sensing neurological brain signals. In other examples, the same electrodes may be used to deliver electrical stimulation and sense brain signals. However, this configuration would require the system to switch between stimulation generation and sensing circuitry and may reduce the time the system can sense brain signals.

Electrical stimulation generated by IMD 106 may be configured to manage a variety of disorders and conditions. In some examples, the stimulation generator of IMD 106 is configured to generate and deliver electrical stimulation pulses to patient 112 via electrodes of a selected stimulation electrode combination. However, in other examples, the stimulation generator of IMD 106 may be configured to generate and deliver a continuous wave signal, e.g., a sine wave or triangle wave. In either case, a stimulation generator within IMD 106 may generate the electrical stimulation therapy for DBS according to a therapy program that is selected at that given time in therapy. In examples in which IMD 106 delivers electrical stimulation in the form of stimulation pulses, a therapy program may include a set of therapy parameter values (e.g., stimulation parameters), such as a stimulation electrode combination for delivering stimulation to patient 112, pulse frequency, pulse width, and a current or voltage amplitude of the pulses. As previously indicated, the electrode combination may indicate the specific electrodes 116, 118 that are selected to deliver stimulation signals to tissue of patient 112 and the respective polarities of the selected electrodes. IMD 106 may deliver electrical stimulation intended to contribute to a therapeutic effect. In some examples, IMD 106 may also, or alternatively, deliver electrical stimulation intended to be sensed by other electrodes and/or elicit a physiological response, such as an evoked compound action potential (ECAP), that can be sensed by electrodes.

IMD 106 may be implanted within a subcutaneous pocket above the clavicle, or, alternatively, on or within cranium 122 or at any other suitable site within patient 112. Generally, IMD 106 is constructed of a biocompatible material that resists corrosion and degradation from bodily fluids. IMD 106 may comprise a hermetic housing to substantially enclose components, such as a processor, therapy module, and memory.

As shown in FIG. 1, implanted lead extension 110 is coupled to IMD 106 via connector 108 (also referred to as a connector block or a header of IMD 106). In the example of FIG. 1, lead extension 110 traverses from the implant site of IMD 106 and along the neck of patient 112 to cranium 122 of patient 112 to access brain 120. In the example shown in FIG. 1, leads 114A and 114B (collectively "leads 114") are implanted within the right and left hemispheres, respectively, of patient 112 in order deliver electrical stimulation to one or more regions of brain 120, which may be selected based on the patient condition or disorder controlled by therapy system 100. The specific target tissue site and the stimulation electrodes used to deliver stimulation to the target tissue site, however, may be selected, e.g., according to the identified patient behaviors and/or other sensed patient parameters. Other implant sites for lead 114 and IMD 106 are contemplated. For example, IMD 106 may be implanted on or within cranium 122, in some examples. Or leads 114 may be implanted within the same hemisphere or IMD 106 may be coupled to a single lead implanted in a single hemisphere. Although leads 114 may have ring electrodes at different longitudinal positions as shown in FIG. 1, leads 114 may have electrodes disposed at different positions around the perimeter of the lead (e.g., different circumferential positions for a cylindrical shaped lead).

Leads 114 illustrate an example lead set that includes axial leads carrying ring electrodes disposed at different axial positions (or longitudinal positions). In other examples, leads may be referred to as "paddle" leads carrying planar arrays of electrodes on one side of the lead structure. In addition, as described herein, complex lead array geometries may be used in which electrodes are disposed at different respective longitudinal positions and different positions around the perimeter of the lead.

Although leads 114 are shown in FIG. 1 as being coupled to a common lead extension 110, in other examples, leads 114 may be coupled to IMD 106 via separate lead extensions or directly to connector 108. Leads 114 may be positioned to deliver electrical stimulation to one or more target tissue sites within brain 120 to manage patient symptoms associated with a movement disorder or other neurological disorder of patient 112. Leads 114 may be implanted to position electrodes 116, 118 at desired locations of brain 120 through respective holes in cranium 122. Leads 114 may be placed at any location within brain 120 such that electrodes 116, 118 are capable of providing electrical stimulation to target tissue sites within brain 120 during treatment. For example, electrodes 116, 118 may be surgically implanted under the dura mater of brain 120 or within the cerebral cortex of brain 120 via a burr hole in cranium 122 of patient 112, and electrically coupled to IMD 106 via one or more leads 114.

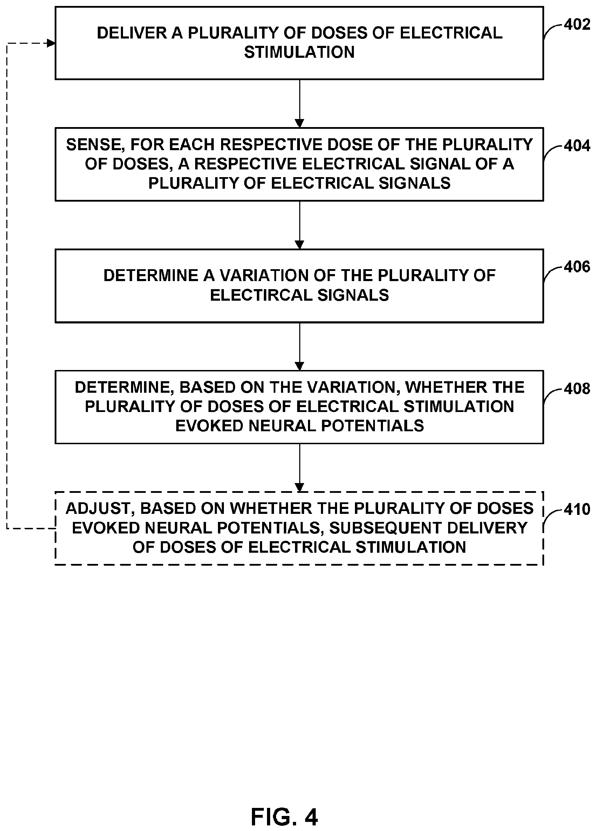

In the example shown in FIG. 1, electrodes 116, 118 of leads 114 are shown as ring electrodes. Ring electrodes may be used in DBS applications because ring electrodes are relatively simple to program and are capable of delivering an electrical field to any tissue adjacent to electrodes 116, 118. In other examples, electrodes 116, 118 may have different configurations. For example, in some examples, at least some of the electrodes 116, 118 of leads 114 may have a complex electrode array geometry that is capable of producing shaped electrical fields. The complex electrode array geometry may include multiple electrodes (e.g., partial ring or segmented electrodes) around the outer perimeter of each lead 114, rather than one ring electrode, such as shown in FIGS. 4A and 4B. In this manner, electrical stimulation may be directed in a specific direction from leads 114 to enhance therapy efficacy and reduce possible adverse side effects from stimulating a large volume of tissue. In some examples, a housing of IMD 106 may include one or more stimulation and/or sensing electrodes. In alternative examples, leads 114 may have shapes other than elongated cylinders as shown in FIG. 1. For example, leads 114 may be paddle leads, spherical leads, bendable leads, or any other type of shape effective in treating patient 112 and/or minimizing invasiveness of leads 114.

In the example shown in FIG. 1, IMD 106 includes a memory to store a plurality of therapy programs that each define a set of therapy parameter values. In some examples, IMD 106 may select a therapy program from the memory based on various parameters, such as sensed patient parameters and the identified patient behaviors. IMD 106 may generate electrical stimulation based on the selected therapy program to manage the patient symptoms associated with a movement disorder.

External programmer 104 wirelessly communicates with IMD 106 as needed to provide or retrieve therapy information. Programmer 104 is an external computing device that the user, e.g., a clinician and/or patient 112, may use to communicate with IMD 106. For example, programmer 104 may be a clinician programmer that the clinician uses to communicate with IMD 106 and program one or more therapy programs for IMD 106. Alternatively, programmer 104 may be a patient programmer that allows patient 112 to select programs and/or view and modify therapy parameters. The clinician programmer may include more programming features than the patient programmer. In other words, more complex or sensitive tasks may only be allowed by the clinician programmer to prevent an untrained patient from making undesirable changes to IMD 106. Programmer 104 may enter a new programming session for the user to select new stimulation parameters for subsequent therapy.

When programmer 104 is configured for use by the clinician, programmer 104 may be used to transmit initial programming information to IMD 106. This initial information may include hardware information, such as the type of leads 114 and the electrode arrangement, the position of leads 114 within brain 120, the configuration of electrode array 116, 118, initial programs defining therapy parameter values, and any other information the clinician desires to program into IMD 106. Programmer 104 may also be capable of completing functional tests (e.g., measuring the impedance of electrodes 116, 118 of leads 114). In some examples, programmer 104 may receive sensed signals or representative information and perform the same techniques and functions attributed to IMD 106 herein. In other examples, a remote server (e.g., a standalone server or part of a cloud service) may perform the functions attributed to IMD 106, programmer 104, or any other devices described herein.

The clinician may also store therapy programs within IMD 106 with the aid of programmer 104. During a programming session, the clinician may determine one or more therapy programs that may provide efficacious therapy to patient 112 to address symptoms associated with the patient condition, and, in some cases, specific to one or more different patient states, such as a sleep state, movement state or rest state. For example, the clinician may select one or more stimulation electrode combination with which stimulation is delivered to brain 120. During the programming session, the clinician may evaluate the efficacy of the specific program being evaluated based on feedback provided by patient 112 or based on one or more physiological parameters of patient 112 (e.g., muscle activity, muscle tone, rigidity, tremor, etc.). Alternatively, identified patient behavior from video information may be used as feedback during the initial and subsequent programming sessions. Programmer 104 may assist the clinician in the creation/identification of therapy programs by providing a methodical system for identifying potentially beneficial therapy parameter values.

Programmer 104 may also be configured for use by patient 112. When configured as a patient programmer, programmer 104 may have limited functionality (compared to a clinician programmer) in order to prevent patient 112 from altering critical functions of IMD 106 or applications that may be detrimental to patient 112. In this manner, programmer 104 may only allow patient 112 to adjust values for certain therapy parameters or set an available range of values for a particular therapy parameter.

Programmer 104 may also provide an indication to patient 112 when therapy is being delivered, when patient input has triggered a change in therapy or when the power source within programmer 104 or IMD 106 needs to be replaced or recharged. For example, programmer 112 may include an alert LED, may flash a message to patient 112 via a programmer display, or generate an audible sound or somatosensory cue to confirm patient input was received, e.g., to indicate a patient state or to manually modify a therapy parameter.

Therapy system 100 may be implemented to provide chronic stimulation therapy to patient 112 over the course of several months or years. However, system 100 may also be employed on a trial basis to evaluate therapy before committing to full implantation. If implemented temporarily, some components of system 100 may not be implanted within patient 112. For example, patient 112 may be fitted with an external medical device, such as a trial stimulator, rather than IMD 106. The external medical device may be coupled to percutaneous leads or to implanted leads via a percutaneous extension. If the trial stimulator indicates DBS system 100 provides effective treatment to patient 112, the clinician may implant a chronic stimulator within patient 112 for relatively long-term treatment.

Although IMD 106 is described as delivering electrical stimulation therapy to brain 120, IMD 106 may be configured to direct electrical stimulation to other anatomical regions of patient 112 in other examples. In other examples, system 100 may include an implantable drug pump in addition to, or in place of, IMD 106. Further, an IMD may provide other electrical stimulation such as spinal cord stimulation (e.g., to treat a movement disorder and/or other condition).

As discussed above, IMD 106 may sense neurological signals (e.g., electrical signals) of patient 112. For instance, circuitry of IMD 106 may sense a differential voltage level across two electrodes of leads 114 or sense a differential voltage level across an electrode of a lead of leads 114 and a case (i.e.., "can") electrode of IMD 106. In some situations, it may be desirable to sense neural potentials evoked in response to electrical stimulation (e.g., for use as a control signal in closed loop stimulation). Evoked neural potentials evoked can take a long time to evolve (e.g., 30 milliseconds, 40 milliseconds) in response to electrical stimulation (e.g., there may be a temporal gap between delivery of electrical stimulation and a neural potential resulting from the delivery). For instance, after IMD 106 delivers a dose of electrical stimulation, at least 30 milliseconds may elapse between delivery of the dose of electrical stimulation and evocation of a neural potential. By contrast, stimulation paradigms may have relatively high stimulation rates (e.g., 150 Hz for deep brain stimulation (DBS)). Attempting to sense such delayed neural potentials in the presence of high stimulation rates with an IMD may be difficult. For instance, if the IMD were to sense electrical signals and attempt to directly detect neural responses in the electrical signals, the neural responses may be masked by large stimulation artifacts which may make it difficult for the IMD to capture their features or even detect their presence, especially in cases when the bandwidth (number of samples per seconds of data captured) is limited.

In accordance with one or more techniques of this disclosure, as opposed to directly using sensed electrical signals (e.g., voltage measurements) of evoked neural potentials in response to electrical stimulation, IMD 106 may utilize variations of the sensed electrical signals (e.g., variations of a characteristic, such as amplitude, of the sensed electrical signals). The stimulation delivery may be nearly identical every time. However, as neural potentials are biological, the evoked neural potentials may vary slightly every time. This variation may become apparent when examining measurements captured after several stimulation deliveries (e.g., several doses, each of which may include several pulses). IMD 106 may examine variability across time to distinguish second-order potentials from artifacts and immediate synchronized responses. Increases in variation of the response may imply second order responses to neural stimulation. As such, IMD 106 may determine, based on variations of the plurality of electrical signals, whether a plurality of doses of electrical stimulation evoked neural potentials.

IMD 106 may utilize the results of the variation analysis in any of a variety of ways. As one example, IMD 106 may perform closed-loop stimulation based on results of the sensing. For instance, where the doses of electrical stimulation did not evoke neural potentials, IMD 106 may adjust one or more parameters of subsequent electrical stimulation to increase a probability that the subsequent electrical stimulation will evoke neural potentials. As another example, IMD 106 may output an indication (e.g., to an external device) as to whether the doses of electrical stimulation evoked neural potentials.

The architecture of system 100 illustrated in FIG. 1 is shown as an example. The techniques as set forth in this disclosure may be implemented in the example system 100 of FIG. 1, as well as other types of systems not described specifically herein. Nothing in this disclosure should be construed so as to limit the techniques of this disclosure to the example architecture illustrated by FIG. 1. As one example, the analysis of the sensed electrical signals may be performed by a device other than IMD 106, such as programmer 104 or a remote cloud server application.

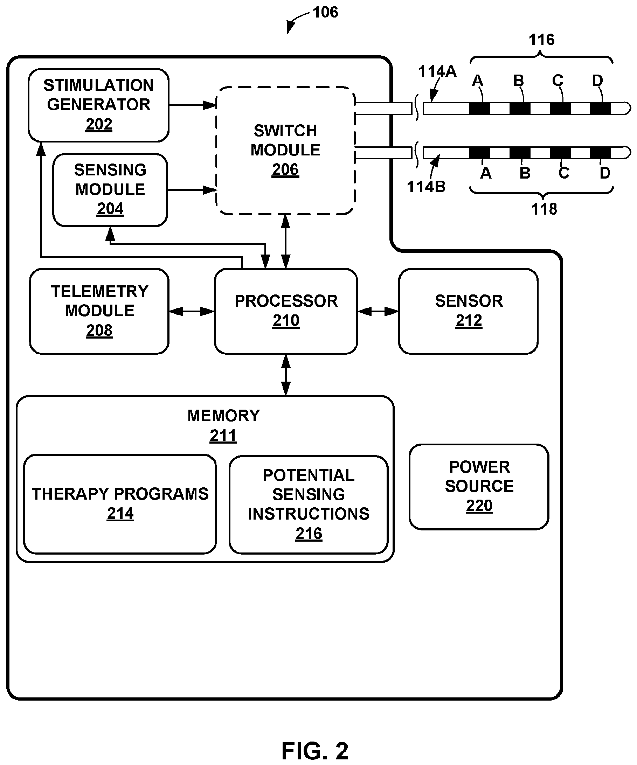

FIG. 2 is a block diagram of the example IMD 106 of FIG. 1 for delivering DBS therapy. In the example shown in FIG. 2, IMD 106 includes processor 210, memory 211, stimulation generator 202, sensing module 204, switch module 206, telemetry module 208, sensor 212, and power source 220. Each of these modules may be or include electrical circuitry configured to perform the functions attributed to each respective module. For example, processor 210 may include processing circuitry, switch module 206 may include switch circuitry, sensing module 204 may include sensing circuitry, and telemetry module 208 may include telemetry circuitry. Switch module 204 may not be necessary for multiple current source and sink configurations. For instance, IMD 106 may include a plurality of current sources and current sinks such that multiplexing via a switch module may not be used. Memory 211 may include any volatile or non-volatile media, such as a random-access memory (RAM), read only memory (ROM), non-volatile RAM (NVRAM), electrically erasable programmable ROM (EEPROM), flash memory, and the like. Memory 211 may store computer-readable instructions that, when executed by processor 210, cause IMD 106 to perform various functions. Memory 211 may be a storage device or other non-transitory medium.

In the example shown in FIG. 2, memory 211 stores therapy programs 214 that include respective stimulation parameter sets that define therapy. Each stored therapy program 214 defines a particular set of electrical stimulation parameters (e.g., a therapy parameter set), such as a stimulation electrode combination, electrode polarity, current or voltage amplitude, pulse width, and pulse rate. In some examples, individual therapy programs may be stored as a therapy group, which defines a set of therapy programs with which stimulation may be generated. The stimulation signals defined by the therapy programs of the therapy group may be delivered together on an overlapping or nonoverlapping (e.g., time-interleaved) basis. Memory 211 may also include potential sensing instructions 216 that define the process by which processor 210 determines whether neural potentials have been evoked.

Stimulation generator 202, under the control of processor 210, generates stimulation signals for delivery to patient 112 via selected combinations of electrodes 116, 118. An example range of electrical stimulation parameters believed to be effective in DBS to manage a movement disorder of a patient include:
1. Pulse Rate, i.e., Frequency: between approximately 0.1 Hertz and approximately 500 Hertz, such as between approximately 0.1 to 10 Hertz, approximately 40 to 185 Hertz (Hz), or such as approximately 140 Hz.
2. In the case of a voltage controlled system, Voltage Amplitude: between approximately 0.1 volts and approximately 50 volts, such as between approximately 2 volts and approximately 3 volts.
3. In the alternative case of a current controlled system, Current Amplitude: between approximately 0.2 milliamps to approximately 100 milliamps, such as between approximately 1.3 milliamps and approximately 2.0 milliamps.
4. Pulse Width: between approximately 10 microseconds and approximately 5000 microseconds, such as between approximately 100 microseconds and approximately 1000 microseconds, or between approximately 180 microseconds and approximately 450 microseconds.

Accordingly, in some examples, stimulation generator 202 generates electrical stimulation signals in accordance with the electrical stimulation parameters noted above. Other ranges of therapy parameter values may also be useful, and may depend on the target stimulation site within patient 112. While stimulation pulses are described, stimulation signals may be of any form, such as continuous-time signals (e.g., sine waves) or the like. Stimulation signals configured to elicit ECAPs or other evoked physiological signals may be similar or different from the above parameter value ranges.

Processor 210 may include fixed function processing circuitry and/or programmable processing circuitry, and may comprise, for example, any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processor 210 herein, and may be embodied as firmware, hardware, software or any combination thereof. Processor 210 may control stimulation generator 202 according to therapy programs 214 stored in memory 211 to apply particular stimulation parameter values specified by one or more of programs, such as voltage amplitude or current amplitude, pulse width, or pulse rate.

In the example shown in FIG. 2, the set of electrodes 116 includes electrodes 116A, 116B, 116C, and 116D, and the set of electrodes 118 includes electrodes 118A, 118B, 118C, and 118D. Processor 210 also controls switch module 206 to apply the stimulation signals generated by stimulation generator 202 to selected combinations of electrodes 116, 118. In particular, switch module 204 may couple stimulation signals to selected conductors within leads 114, which, in turn, deliver the stimulation signals across selected electrodes 116, 118. Switch module 206 may be a switch array, switch matrix, multiplexer, or any other type of switching module configured to selectively couple stimulation energy to selected electrodes 116, 118 and to selectively sense neurological brain signals with selected electrodes 116, 118. Hence, stimulation generator 202 is coupled to electrodes 116, 118 via switch module 206 and conductors within leads 114. In some examples, however, IMD 106 does not include switch module 206.

Stimulation generator 202 may be a single channel or multi-channel stimulation generator. In particular, stimulation generator 202 may be capable of delivering a single stimulation pulse, multiple stimulation pulses, or a continuous signal at a given time via a single electrode combination or multiple stimulation pulses at a given time via multiple electrode combinations. In some examples, however, stimulation generator 202 and switch module 206 may be configured to deliver multiple channels on a time-interleaved basis. For example, switch module 206 may serve to time divide the output of stimulation generator 202 across different electrode combinations at different times to deliver multiple programs or channels of stimulation energy to patient 112. In some examples, stimulation generator 202 may comprise multiple voltage or current sources and sinks that are coupled to respective electrodes to drive the electrodes as cathodes or anodes. In this example, IMD 106 may not require the functionality of switch module 206 for time-interleaved multiplexing of stimulation via different electrodes.

Electrodes 116, 118 on respective leads 114 may be constructed of a variety of different designs. For example, one or both of leads 114 may include two or more electrodes at each longitudinal location along the length of the lead, such as multiple electrodes at different perimeter locations around the perimeter of the lead at each of the locations A, B, C, and D. On one example, the electrodes may be electrically coupled to switch module 206 via respective wires that are straight or coiled within the housing the lead and run to a connector at the proximal end of the lead. In another example, each of the electrodes of the lead may be electrodes deposited on a thin film. The thin film may include an electrically conductive trace for each electrode that runs the length of the thin film to a proximal end connector. The thin film may then be wrapped (e.g., a helical wrap) around an internal member to form the lead 114. These and other constructions may be used to create a lead with a complex electrode geometry.

Although sensing module 204 is incorporated into a common housing with stimulation generator 202 and processor 210 in FIG. 2, in other examples, sensing module 204 may be in a separate housing from IMD 106 and may communicate with processor 210 via wired or wireless communication techniques. Example neurological brain signals include, but are not limited to, a signal generated from local field potentials (LFPs) within one or more regions of brain 28. EEG and ECoG signals are examples of local field potentials (LFPs) that may be measured within brain 120. However, local field potentials may include a broader genus of electrical signals within brain 120 of patient 112. Instead of, or in addition to, LFPs, IMD 106 may be configured to detect patterns of single-unit activity and/or multi-unit activity. IMD 106 may sample this activity at rates above 1,000 Hz, and in some examples within a frequency range of 6,000 Hz to 40,000 Hz. IMD 106 may identify the wave-shape of single units and/or an envelope of unit modulation that may be features used to differentiate or rank electrodes. In some examples, this technique may include phase-amplitude coupling to the envelope or to specific frequency bands in the LFPs sensed from the same or different electrodes.

Sensor 212 may include one or more sensing elements that sense values of a respective patient parameter. For example, sensor 212 may include one or more accelerometers, optical sensors, chemical sensors, temperature sensors, pressure sensors, or any other types of sensors. Sensor 212 may output patient parameter values that may be used as feedback to control delivery of therapy. IMD 106 may include additional sensors within the housing of IMD 106 and/or coupled via one of leads 114 or other leads. In addition, IMD 106 may receive sensor signals wirelessly from remote sensors via telemetry module 208, for example. In some examples, one or more of these remote sensors may be external to patient (e.g., carried on the external surface of the skin, attached to clothing, or otherwise positioned external to the patient).

Telemetry module 208 supports wireless communication between IMD 106 and an external programmer 104 or another computing device under the control of processor 210. Processor 210 of IMD 106 may receive, as updates to programs, values for various stimulation parameters such as magnitude and electrode combination, from programmer 104 via telemetry module 208. The updates to the therapy programs may be stored within therapy programs 214 portion of memory 211. In addition, processor 210 may control telemetry module 208 to transmit alerts or other information to programmer 104 that indicate a lead moved with respect to tissue. Telemetry module 208 in IMD 106, as well as telemetry modules in other devices and systems described herein, such as programmer 104, may accomplish communication by radiofrequency (RF) communication techniques. In addition, telemetry module 208 may communicate with external medical device programmer 104 via proximal inductive interaction of IMD 106 with programmer 104. Accordingly, telemetry module 208 may send information to external programmer 104 on a continuous basis, at periodic intervals, or upon request from IMD 106 or programmer 104. For instance, telemetry module 208 may send representations of sensed electrical signals to external programmer 104.

Power source 220 delivers operating power to various components of IMD 106. Power source 220 may include a small rechargeable or non-rechargeable battery and a power generation circuit to produce the operating power. Recharging may be accomplished through proximal inductive interaction between an external charger and an inductive charging coil within IMD 220. In some examples, power requirements may be small enough to allow IMD 220 to utilize patient motion and implement a kinetic energyscavenging device to trickle charge a rechargeable battery. In other examples, traditional batteries may be used for a limited period of time.

According to the techniques of the disclosure, processor 210 of IMD 106 delivers, via electrodes 116, 118 interposed along leads 114 (and optionally switch module 206), electrical stimulation therapy to patient 112. The DBS therapy is defined by one or more therapy programs 214 having one or more parameters stored within memory 211. For example, the one or more parameters include a current amplitude (for a current-controlled system) or a voltage amplitude (for a voltage-controlled system), a pulse rate or frequency, and a pulse width, or quantity of pulses per cycle. In examples where the electrical stimulation is delivered according to a "burst" of pulses, or a series of electrical pulses defined by an "on-time" and an "off-time," the one or more parameters may further define one or more of a number of pulses per burst, an on-time, and an off-time.

As noted above, sensing module 204 may sense electrical signals via electrodes of leads 114. However, in some circumstances, delivery of electrical stimulation (e.g., by stimulation generator 202 and electrodes of leads 114) may introduce artifacts, referred to as stimulation artifacts, in the sensed electrical signals. In general, it may be desirable for IMD 106 to mitigate the impact of stimulation artifacts.

In addition to the desire to mitigate stimulation artifacts, other aspects may complicate the sensing of electrical signals. As one example, timing constraints (e.g., continuous firmware management of stimulation, and/or data acquisition and transmission) may complicate the sensing of electrical signals. As another example, hardware constraints (e.g., amplifier timing, signal processing latency, and/or telemetry latency) may complicate the sensing of electrical signals.

Memory 211 may also include potential sensing instructions 216 that define the process by which processor 210 determines whether delivered electrical stimulation evoked neural potential(s). In accordance with one or more techniques of this disclosure, processor 210 may execute potential sensing instructions 216 to determine a variation (e.g., a standard deviation or a variance) of a plurality of electrical signals (e.g., variation of an amplitude of the plurality of electrical signals). Each electrical signal of the plurality of electrical signals may correspond to a respective signal sensed by sensing module 204 commensurate with delivery of a dose of electrical stimulation of a plurality of doses of electrical stimulation.

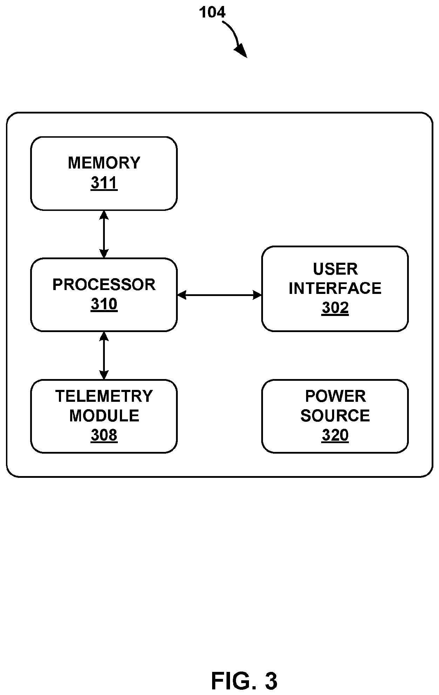

FIG. 3 is a block diagram of the external programmer 104 of FIG. 1 for controlling delivery of DBS therapy according to an example of the techniques of the disclosure. Although programmer 104 may generally be described as a hand-held device, programmer 104 may be a larger portable device or a more stationary device. In some examples, programmer 104 may be referred to as a tablet computing device. In addition, in other examples, programmer 104 may be included as part of a bed-side monitor, an external charging device or include the functionality of an external charging device. As illustrated in FIG. 3, programmer 104 may include a processor 310, memory 311, user interface 302, telemetry module 308, and power source 320. Memory 311 may store instructions that, when executed by processor 310, cause processor 310 and external programmer 104 to provide the functionality ascribed to external programmer 104 throughout this disclosure. Each of these components, or modules, may include electrical circuitry that is configured to perform some or all of the functionality described herein. For example, processor 310 may include processing circuitry configured to perform the processes discussed with respect to processor 310.

In general, programmer 104 comprises any suitable arrangement of hardware, alone or in combination with software and/or firmware, to perform the techniques attributed to programmer 104, and processor 310, user interface 302, and telemetry module 308 of programmer 104. In various examples, programmer 104 may include one or more processors, which may include fixed function processing circuitry and/or programmable processing circuitry, as formed by, for example, one or more microprocessors, DSPs, ASICs, FPGAs, or any other equivalent integrated or discrete logic circuitry, as well as any combinations of such components. Programmer 104 also, in various examples, may include a memory 311, such as RAM, ROM, PROM, EPROM, EEPROM, flash memory, a hard disk, a CD-ROM, comprising executable instructions for causing the one or more processors to perform the actions attributed to them. Moreover, although processor 310 and telemetry module 308 are described as separate modules, in some examples, processor 310 and telemetry module 308 may be functionally integrated with one another. In some examples, processor 310 and telemetry module 308 correspond to individual hardware units, such as ASICs, DSPs, FPGAs, or other hardware units.

Memory 311 (e.g., a storage device) may store instructions that, when executed by processor 310, cause processor 310 and programmer 104 to provide the functionality ascribed to programmer 104 throughout this disclosure. For example, memory 311 may include instructions that cause processor 310 to obtain a parameter set from memory, select a spatial electrode movement pattern, provide an interface that recommends or otherwise facilitates parameter value selection, or receive a user input and send a corresponding command to IMD 106, or instructions for any other functionality. In addition, memory 311 may include a plurality of programs, where each program includes a parameter set that defines stimulation therapy.

User interface 302 may include a button or keypad, lights, a speaker for voice commands, a display, such as a liquid crystal (LCD), light-emitting diode (LED), or organic light-emitting diode (OLED). In some examples the display may be a touch screen. User interface 302 may be configured to display any information related to the delivery of stimulation therapy, identified patient behaviors, sensed patient parameter values, patient behavior criteria, or any other such information. User interface 302 may also receive user input via user interface 302. The input may be, for example, in the form of pressing a button on a keypad or selecting an icon from a touch screen.

Telemetry module 308 may support wireless communication between IMD 106 and programmer 104 under the control of processor 310. Telemetry module 308 may also be configured to communicate with another computing device via wireless communication techniques, or direct communication through a wired connection. In some examples, telemetry module 308 provides wireless communication via an RF or proximal inductive medium. In some examples, telemetry module 308 includes an antenna, which may take on a variety of forms, such as an internal or external antenna. In some examples, IMD 106 and/or programmer 104 may communicate with remote servers via one or more cloud-services in order to deliver and/or receive information between a clinic and/or programmer.

Examples of local wireless communication techniques that may be employed to facilitate communication between programmer 104 and IMD 106 include RF communication according to the 802.11 or Bluetooth specification sets or other standard or proprietary telemetry protocols. In this manner, other external devices may be capable of communicating with programmer 104 without needing to establish a secure wireless connection. As described herein, telemetry module 308 may be configured to transmit a spatial electrode movement pattern or other stimulation parameter values to IMD 106 for delivery of stimulation therapy.

According to the techniques of the disclosure, in some examples, processor 310 of external programmer 104 may analyze sensed electrical signals to determine whether doses of electrical stimulation evoked neural potentials. For instance, processor 310 may receive, via telemetry module 308, representations of a plurality of sensed electrical signals (e.g., digital representations of sensed analog electrical signals). Processor 310 may execute instructions, similar to potential sensing instructions 216, to analyze the representations of a plurality of sensed electrical signals to determine whether neural potentials were evoked. For instance, processor 310 may calculate a variation of the plurality of electrical signals and determine, based on the variation, whether neural potentials were evoked.

Regardless of whether the determination of neural potential evocation was performed at programmer 104, IMD 106, or another device, programmer 104 may, output a result of the determination. For instance, processors 310 may cause user interface 302 to output a graphical user interface (GUI) that includes at least an indication as to whether neural potentials were evoked.

FIG. 4 is a flowchart illustrating an example technique for sensing of evoked neural potentials, in accordance with one or more techniques of this disclosure. For purposes of explanation, the technique of FIG. 4 is described as being performed by IMD 106. However, all or portions of the technique of FIG. 4 may be performed other devices.

IMD 106 may deliver a plurality of doses of electrical stimulation to a patient (402). For instance, stimulation generator 202 may generate, and deliver via one or more of electrodes 116 and 118, N doses of electrical stimulation to patient 112. Stimulation generator 202 may generate and deliver the plurality of doses with a set of stimulation parameters (e.g., at a particular pulse rate, amplitude (e.g., current or voltage), and pulse width). In some examples, the pulse rate may be greater than or equal to 100 Hz (e.g., from 75 Hz to 150 Hz). Each dose of electrical stimulation may include one or more pulses of electrical stimulation (e.g., delivered with a set of stimulation parameters such as amplitude, pulse width, duty cycle, etc.).

IMD 106 may sense, for each respective dose of the plurality of doses, a respective electrical signal of a plurality of electrical signals (404). For instance, sensing module 204 may sense *N* electrical signals. Each of the *N* senses electrical signals may correspond to a particular dose of electrical stimulation of the N doses of electrical stimulation. For instance, a particular sensed electrical signal may represent electrical activity temporally proximate to a particular dose of electrical stimulation.

IMD 106 may determine a variation of the plurality of sensed electrical signals (406). For instance, processor 210 may execute potential sensing instructions 216 to analyze the plurality of electrical signals to calculate the variation signal. In some examples, the variation may be a standard deviation of the plurality of electrical signals. In some examples, the variation may be a variance of the plurality of electrical signals.

IMD 106 may determine, based on the variation, whether the plurality of doses of electrical stimulation evoked neural potentials (408). The evoked neural potentials may be potentials produced by the nervous system in response to the doses of electrical stimulation (e.g., may be different that an intrinsic sensed potential). For instance, processor 210 may execute potential sensing instructions 216 to analyze the variation signal for the presence of peak values. Where a peak value of the variation signal is more than a threshold amount greater than an average value of the variation signal, IMD 106 may determine that the plurality of doses of electrical stimulation did evoke neural potentials or a change in evoked neural potentials. Similarly, where the peak value of the variation signal is not more than the threshold amount (e.g., 10%, 20%, 30% of the average value of the variation signal) greater than the average value of the variation signal, IMD 106 may determine that the plurality of doses of electrical stimulation did not evoke neural potentials.

In some examples, IMD 106 may perform pre-processing of the sensed electrical signals (e.g., prior to variation determination). For instance, processor 210 may perform one or more of outlier removal, artifact removal, temporal filtering, and/or spatial filtering. As one example, processor 210 may "throw out" or otherwise remove electrical signals that have large potentials (e.g., due to movements). As another example, processor 210 may subtract drifts in the data and DC shifts. As another example, processors 210 may perform smoothing.

As discussed above, determining the evocation of neural potentials in response to electrical stimulation using the variation of sensed signals may provide various advantages. For instance, the stimulation delivery may be nearly identical every time (e.g., each dose of the plurality of doses may be delivered with similar or identical stimulation parameters). However, as neural potentials are biological, the evoked neural potentials may vary slightly every time, e.g., according to patient activity, variation in a disease or disorder, or other factors. As such, neural potentials (or features of neural potentials) may be substantially more apparent in a variation signal than in the original sampled signal. In this way, the techniques of this disclosure may provide for improved detection of evoked neural potentials, i.e., neural potentials that are evoked by tissue in response to stimulation versus signals sensed when stimulation does not cause the tissue to evoke neural potentials.

Occasionally it is useful to distinguish direct neural responses (e.g. responses of neurons to electrical fields generated by the stimulator) from secondary responses (responses evoked through directly excited neurons transmitting their excitation through synapses to other neurons). In addition, through several synaptic connections, neurons may form a network whereby information flows from one part of the nervous system to another, and then flows back.

For example, in Parkinson disease, several nuclei send information to the motor cortex, and then receive information back. Stimulation which evokes responses of this network may be more effective than stimulation that just evokes a localized neural structure. Because potentials of synaptic transmission involve networks of cells which may be otherwise influenced, the signals can vary more from stimulation pulse to stimulation pulse compared to direct electrical stimulation. For this reason, one expects the variation of the network response to be larger compared to the direct response. Increased variation may therefore be a specifically sensitive to evoking such neural networks and thereby identifying effective stimulation paradigms.

IMD 106 may perform one or more actions based on whether the plurality of doses of electrical stimulation evoked neural potentials. As one example, IMD 106 may adjust subsequent delivery of doses of electrical stimulation (410). For instance, where IMD 106 delivered the plurality of doses of electrical stimulation with a first set of stimulation parameters (402), IMD 106 may determine, based on whether the plurality of doses evoked neural potentials, a second set of stimulation parameters. In some examples, to determine the second set of stimulation parameters, IMD 106 may determine, responsive to determining that the plurality of doses did not evoke neural potentials in the patient, the second set of stimulation parameters to have an increased likelihood of evoking neural potentials (e.g., to include an amplitude that is greater than an amplitude of the first set of stimulation parameters). In some examples, such as there the plurality of doses of electrical stimulation did evoke neural potentials, IMD 106 may not adjust the parameters (e.g., maintain the first set of stimulation parameters). In this way, the techniques of this disclosure may provide for closed-loop stimulation based on evoked neural potentials.

As another example, IMD 106 may output an indication of whether the plurality of doses of electrical stimulation evoked neural potentials to an external device. For instance, IMD 106 may output, via telemetry module 208, a signal to programmer 104 that indicates whether the plurality of doses of electrical stimulation evoked neural potentials to an external device. The external device may output a notification and/or output/generate a report for a user that includes the indication.

In some examples, IMD 106 (or another device) may track disease progression. For instance, at a first time, IMD 106 may deliver a first plurality of doses of electrical stimulation with a first set of stimulation parameters. IMD 106 may determine, based on whether the first plurality of doses of electrical stimulation evoked neural potentials in the patient, a disease state of the patient. Then, at a second time that is later than the first time, IMD 106 may deliver a second plurality of doses of electrical stimulation with the first set of stimulation parameters (i.e., the same set of stimulation parameters). IMD 106 may determine, based on whether the second plurality of doses of electrical stimulation evoked neural potentials in the patient, whether the disease state of the patient has changed. For instance, if the first plurality of doses did evoke neural potentials but the second plurality of doses did not, IMD 106 may determine that the disease state of the patient has changed. While described as comparing evocation responses at two different times, the techniques of this disclosure are not so limited. For instance, IMD 106 may track evocation responses at N (where N is greater than or equal to 2) different times to track disease or condition progression. As noted above, progression or trends in the variation measure could be the signal for changing therapy

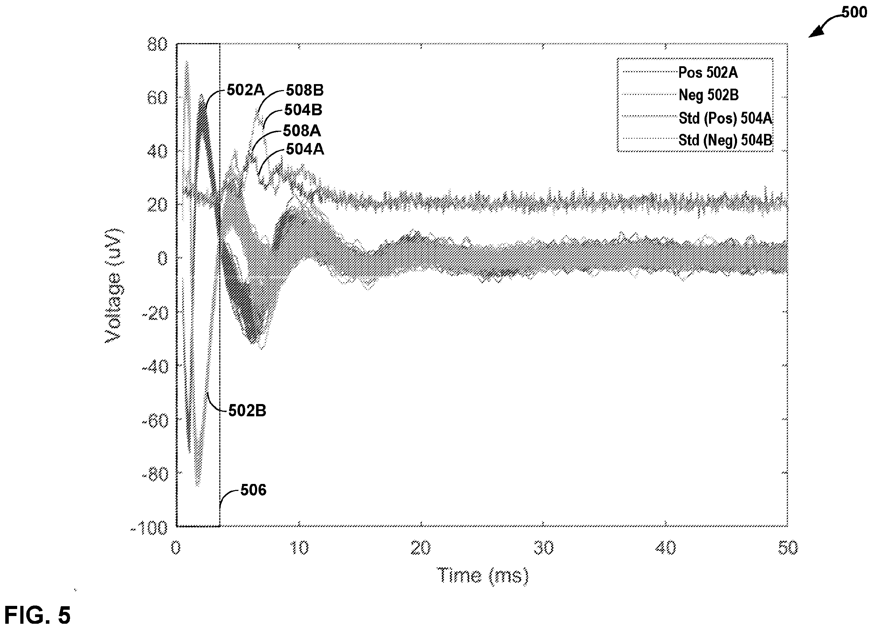

FIG. 5 is a graph illustrating example sensed electrical signals and their variations, in accordance with one or more techniques of this disclosure. The graph of FIG. 5 shows voltage in microvolts over time in milliseconds. As discussed above, IMD 106 may sense a plurality of electrical signals, calculate a variation of the plurality of electrical signals, and determine whether neural potentials have been evoked based on the variation. As shown in FIG. 5, graph 500 includes four signals: positive electrical signal 502A (e.g., a cathodic anodic signal), negative electrical signal 502B (e.g., an anodic cathodic signal), variation signal 504A, and variation signal 504B. Variation signal 504A may be a variance of a voltage level of positive electrical signal 502A. Similarly, variation signal 504B may be a variation of a voltage level negative electrical signal 502B. For ease of illustration, values of variation signals 504A and 504B have been multiplied by 5 in the example of FIG. 5 (e.g., so as not to be overlain on signals 502A and 502B).

As discussed above, stimulation delivery may be nearly identical every time (e.g., each dose of the plurality of doses may be delivered with similar or identical stimulation parameters, such as similar or identical amplitude, pulse width and pulse rate). However, as neural potentials are biological, the evoked neural potentials may vary slightly. In the example of FIG. 5, doses of electrical stimulation may be delivered at time 0 and signals 502A and 502B may represent electrical activity sensed thereafter. As can be seen in FIG. 5, there may be little variation of signal 502A and signal 502B immediately after delivery of electrical stimulation. For instance, during "artifact period" 506, the variations 504A and 504B may be relatively low. However, as can also be seen in FIG. 5, during approximately times 5-8ms after delivery of stimulation, peaks may be observed in the variation signals. For instance, peak 508A may be present in variation signal 504A and peak 508B may be present in variation signal 504B. As discussed above, a device (e.g., IMD 106) may determine whether the delivery of electrical stimulation evoked neural potentials based on values of the peaks.

The techniques described in this disclosure may be implemented, at least in part, in hardware, software, firmware or any combination thereof. For example, various aspects of the described techniques may be implemented within one or more processors, such as fixed function processing circuitry and/or programmable processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

Various examples have been described. These and other examples are within the scope of the following claims.

## Claims

1. A computer-readable storage medium including instructions that, when executed, cause processing circuitry to perform a method, the method comprising:
delivering, by an implantable stimulation device, a plurality of doses of electrical stimulation to a patient;
sensing, by the implantable stimulation device, a plurality of electrical signals wherein the plurality of electrical signals comprises a respective electrical signal for each respective dose of the plurality of doses; and
determining a variation across time based on the plurality of electrical signals;
determining, based on applying a threshold amount to the variation of the plurality of electrical signals, whether the plurality of doses of electrical stimulation evoked neural potentials are indicative of a response of the patient to the plurality of doses of electrical stimulation.

2. The computer-readable storage medium of claim 1, wherein the method further comprises either:
determining the variation as a standard deviation of the plurality of electrical signals; or
determining the variation as a variance of the plurality of electrical signals.

3. The computer-readable storage medium of any of the preceding claims, wherein delivering the plurality of doses of electrical stimulation comprises delivering a first plurality of doses of electrical stimulation with a first set of stimulation parameters, the method further comprising:
determining, based on whether the first plurality of doses of electrical stimulation evoked neural potentials in the patient, a second set of stimulation parameters.

4. The computer-readable storage medium of claim 3, wherein determining the second set of stimulation parameters comprises:
responsive to determining that the first plurality of doses did not evoke neural potentials in the patient, determining the second set of stimulation parameters to include an amplitude that is greater than an amplitude of the first set of stimulation parameters.

5. The computer-readable storage medium of claim 3, wherein the method further comprises:
delivering, by the implantable stimulation device and with the second set of stimulation parameters, a second plurality of doses of electrical stimulation.

6. The computer-readable storage medium of any of the claims 1 or 2, wherein delivering the plurality of doses of electrical stimulation comprises delivering, at a first time, a first plurality of doses of electrical stimulation with a first set of stimulation parameters, the method further comprising:
determining, based on whether the first plurality of doses of electrical stimulation evoked neural potentials in the patient, a disease state of the patient.

7. The computer-readable storage medium of any of the preceding claims, wherein delivering the doses of electrical stimulation comprises:
delivering does of electrical stimulation at a rate that is greater than or equal to 100 Hz.

8. A system comprising:
a memory (211); and
processing circuitry (210) configured to:
cause an implantable stimulation device to deliver a plurality of doses of electrical stimulation to a patient;
receive a plurality of electrical signals, wherein the plurality of electrical signals comprises a respective electrical signal for each respective dose of the plurality of doses; and
determining a variation across time based on the plurality of electrical signals;
determine, based on applying a threshold amount to the variation of the plurality of electrical signals, whether the plurality of doses of electrical stimulation evoked neural potentials are indicative of a response of the patient to the plurality of doses of electrical stimulation.

9. The system of claim 8, wherein the processing circuitry (210) is further configured to either:
determine the variation as a standard deviation of the plurality of electrical signals;
or
determine the variation as a variance of the plurality of electrical signals.

10. The system of any of the claims 8 or 9, wherein, to cause the implantable stimulation device to deliver the plurality of doses of electrical stimulation, the processing circuitry is configured to cause the implantable stimulation device to deliver a first plurality of doses of electrical stimulation with a first set of stimulation parameters, and wherein the processing circuitry is further configured to:
determine, based on whether the first plurality of doses of electrical stimulation evoked neural potentials in the patient, a second set of stimulation parameters.

11. The system of claim 10, wherein, to determine the second set of stimulation parameters, the processing circuitry (210) is configured to:
determine, responsive to determining that the first plurality of doses did not evoke neural potentials in the patient, the second set of stimulation parameters to include an amplitude that is greater than an amplitude of the first set of stimulation parameters.

12. The system of claim 10, wherein the processing circuitry (210) is further configured to:
cause the implantable stimulation device to deliver, with the second set of stimulation parameters, a second plurality of doses of electrical stimulation.

13. The system of any of the claims 8 or 9, wherein, to cause the implantable stimulation device to deliver the plurality of doses of electrical stimulation, the processing circuitry (210) is configured to cause the implantable stimulation device to deliver, at a first time, a first plurality of doses of electrical stimulation with a first set of stimulation parameters, and wherein the processing circuitry (210) is further configured to:
determine, based on whether the first plurality of doses of electrical stimulation evoked neural potentials in the patient, a disease state of the patient.

14. The system of claim 13, wherein the processing circuitry (210) is further configured to:
cause the implantable stimulation device to deliver, at a second time that is after the first time, a second plurality of doses of electrical stimulation with the first set of stimulation parameters; and
determine, based on whether the second plurality of doses of electrical stimulation evoked neural potentials in the patient, whether the disease state of the patient has changed.

15. The system of claim 8, wherein the processing circuitry (210) is further configured to:
adjust, based on whether the plurality of doses of electrical stimulation evoked neural potentials in the patient, subsequent delivery of electrical stimulation to a patient.

## Patentansprüche

1. Computerlesbares Speicherungsmedium, das Anweisungen einschließt, die, wenn sie ausgeführt werden, die Verarbeitungsschaltungsanordnung veranlassen, ein Verfahren durchzuführen, das Verfahren umfassend:
Abgeben, durch eine implantierbare Stimulationsvorrichtung, einer Vielzahl von Dosen elektrischer Stimulation an einen Patienten;
Erfassen, durch die implantierbare Stimulationsvorrichtung, einer Vielzahl von elektrischen Signalen, wobei die Vielzahl von elektrischen Signalen ein jeweiliges elektrisches Signal für jede jeweilige Dosis der Vielzahl von Dosen umfasst; und
Bestimmen einer Variation über die Zeit basierend auf der Vielzahl von elektrischen Signalen;
Bestimmen, basierend auf einem Anwenden eines Schwellenwerts auf die Variation der Vielzahl von elektrischen Signalen, ob die Vielzahl von Dosen elektrischer Stimulation, die neurale Potentiale evoziert haben, auf eine Reaktion des Patienten auf die Vielzahl von Dosen elektrischer Stimulation hinweisen.

2. Computerlesbares Speicherungsmedium nach Anspruch 1, wobei das Verfahren ferner entweder umfasst:
Bestimmen der Variation als eine Standardabweichung der Vielzahl von elektrischen Signalen; oder
Bestimmen der Variation als eine Varianz der Vielzahl von elektrischen Signalen.

3. Computerlesbares Speicherungsmedium nach einem der vorstehenden Ansprüche, wobei
das Abgeben der Vielzahl von Dosen elektrischer Stimulation das Abgeben einer ersten Vielzahl von Dosen elektrischer Stimulation mit einem ersten Satz von Stimulationsparametern umfasst, das Verfahren ferner umfassend:
Bestimmen, basierend darauf, ob die erste Vielzahl von Dosen elektrischer Stimulation neurale Potentiale in dem Patienten evoziert hat, eines zweiten Satzes von Stimulationsparametern.

4. Computerlesbares Speicherungsmedium nach Anspruch 3, wobei das Bestimmen des zweiten Satzes von Stimulationsparametern umfasst:
als Reaktion auf das Bestimmen, dass die erste Vielzahl von Dosen keine neuronalen Potentiale in dem Patienten evoziert hat, Bestimmen des zweiten Satzes von Stimulationsparametern, um eine Amplitude einzuschließen, die größer als eine Amplitude des ersten Satzes von Stimulationsparametern ist.

5. Computerlesbares Medium nach Anspruch 3, wobei das Verfahren ferner umfasst:
Abgeben, durch die implantierbare Stimulationsvorrichtung und mit dem zweiten Satz von Stimulationsparametern, einer zweiten Vielzahl von Dosen elektrischer Stimulation.

6. Computerlesbares Speicherungsmedium nach einem der Ansprüche 1 oder 2, wobei das Abgeben der Vielzahl von Dosen elektrischer Stimulation das Abgeben einer ersten Vielzahl von Dosen elektrischer Stimulation mit einem ersten Satz von Stimulationsparametern zu einem ersten Zeitpunkt umfasst, das Verfahren ferner umfassend:
Bestimmen, basierend darauf, ob die erste Vielzahl von Dosen elektrischer Stimulation neurale Potentiale in dem Patienten evoziert hat, eines Krankheitszustands des Patienten.

7. Computerlesbares Speicherungsmedium nach einem der vorstehenden Ansprüche, wobei das Abgeben der Dosen elektrischer Stimulation umfasst:
Abgeben von Dosen elektrischer Stimulation mit einer Rate, die größer als oder gleich 100 Hz ist.

8. System, umfassend:
einen Speicher (211); und
eine Verarbeitungsschaltungsanordnung (210), die konfiguriert ist zum:
Veranlassen, dass eine implantierbare Stimulationsvorrichtung eine Vielzahl von Dosen elektrischer Stimulation an einen Patienten abgibt;
Empfangen einer Vielzahl von elektrischen Signalen, wobei die Vielzahl von elektrischen Signalen ein jeweiliges elektrisches Signal für jede jeweilige Dosis der Vielzahl von Dosen umfasst; und
Bestimmen einer Variation über die Zeit basierend auf der Vielzahl von elektrischen Signalen;
Bestimmen, basierend auf dem Anwenden eines Schwellenwerts auf die Variation der Vielzahl von elektrischen Signalen, ob die Vielzahl von Dosen elektrischer Stimulation, die neurale Potentiale evoziert haben, auf eine Reaktion des Patienten auf die Vielzahl von Dosen elektrischer Stimulation hinweisen.

9. System nach Anspruch 8, wobei die Verarbeitungsschaltungsanordnung (210) ferner konfiguriert ist entweder zum:
Bestimmen der Variation als eine Standardabweichung der Vielzahl von elektrischen Signalen;
oder
Bestimmen der Variation als eine Varianz der Vielzahl von elektrischen Signalen.

10. System nach einem der Ansprüche 8 oder 9, wobei, um zu veranlassen, dass die implantierbare Stimulationsvorrichtung die Vielzahl von Dosen elektrischer Stimulation abgibt, die Verarbeitungsschaltungsanordnung konfiguriert ist, um zu veranlassen, dass die implantierbare Stimulationsvorrichtung eine erste Vielzahl von Dosen elektrischer Stimulation mit einem ersten Satz von Stimulationsparametern abgibt, und wobei die Verarbeitungsschaltungsanordnung ferner konfiguriert ist zum:
Bestimmen, basierend darauf, ob die erste Vielzahl von Dosen elektrischer Stimulation neurale Potentiale in dem Patienten evoziert hat, eines zweiten Satzes von Stimulationsparametern.

11. System nach Anspruch 10, wobei, um den zweiten Satz von Stimulationsparametern zu bestimmen, die Verarbeitungsschaltungsanordnung (210) konfiguriert ist zum:
Bestimmen, als Reaktion auf das Bestimmen, dass die erste Vielzahl von Dosen keine neuronalen Potentiale in dem Patienten evoziert hat, des zweiten Satzes von Stimulationsparametern, um eine Amplitude einzuschließen, die größer als eine Amplitude des ersten Satzes von Stimulationsparametern ist.

12. System nach Anspruch 10, wobei die Verarbeitungsschaltungsanordnung (210) ferner konfiguriert ist zum:
Veranlassen, dass die implantierbare Stimulationsvorrichtung mit dem zweiten Satz von Stimulationsparametern eine zweite Vielzahl von Dosen elektrischer Stimulation abgibt.

13. System nach einem der Ansprüche 8 oder 9, wobei, um zu veranlassen, dass die implantierbare Stimulationsvorrichtung die Vielzahl von Dosen elektrischer Stimulation abgibt, die Verarbeitungsschaltungsanordnung (210) konfiguriert ist, um zu veranlassen, dass die implantierbare Stimulationsvorrichtung eine erste Vielzahl von Dosen elektrischer Stimulation mit einem ersten Satz von Stimulationsparametern zu einem ersten Zeitpunkt abgibt, und wobei die Verarbeitungsschaltungsanordnung (210) ferner konfiguriert ist zum:
Bestimmen, basierend darauf, ob die erste Vielzahl von Dosen elektrischer Stimulation neurale Potentiale in dem Patienten evoziert hat, eines Krankheitszustands des Patienten.

14. System nach Anspruch 13, wobei die Verarbeitungsschaltungsanordnung (210) ferner konfiguriert ist zum:
Veranlassen, dass die implantierbare Stimulationsvorrichtung zu einem zweiten Zeitpunkt, der nach dem ersten Zeitpunkt liegt, eine zweite Vielzahl von Dosen elektrischer Stimulation mit dem ersten Satz von Stimulationsparametern abgibt; und
Bestimmen, basierend darauf, ob die zweite Vielzahl von Dosen elektrischer Stimulation neurale Potentiale in dem Patienten evoziert hat, ob sich der Krankheitszustand des Patienten verändert hat.

15. System nach Anspruch 8, wobei die Verarbeitungsschaltungsanordnung (210) ferner konfiguriert ist zum:
Anpassen, basierend darauf, ob die Vielzahl von Dosen elektrischer Stimulation neurale Potentiale in dem Patienten evoziert hat, einer nachfolgenden Abgabe von elektrischer Stimulation an einen Patienten.

## Revendications

1. Support de stockage lisible par un ordinateur comportant des instructions qui, lorsqu'elles sont exécutées, amènent le circuit de traitement à exécuter un procédé, le procédé comprenant :
l'administration, par un dispositif de stimulation implantable, d'une pluralité de doses de stimulation électrique à un patient ;
la détection, par le dispositif de stimulation implantable, d'une pluralité de signaux électriques, dans lequel la pluralité de signaux électriques comprend un signal électrique respectif pour chaque dose respective de la pluralité de doses ; et
la détermination d'une variation dans le temps en fonction de la pluralité de signaux électriques ;
la détermination, en fonction de l'application d'une quantité seuil à la variation de la pluralité de signaux électriques, si la pluralité de doses de potentiels neuronaux évoqués par stimulation électrique indiquent une réponse du patient à la pluralité de doses de stimulation électrique.

2. Support de stockage lisible par ordinateur selon la revendication 1, dans lequel le procédé comprend en outre soit :
la détermination de la variation en tant qu'écart type de la pluralité de signaux électriques ; ou
la détermination de la variation en tant que variance de la pluralité de signaux électriques.

3. Support de stockage lisible par ordinateur selon l'une quelconque des revendications précédentes, dans lequel l'administration de la pluralité de doses de stimulation électrique comprend l'administration d'une première pluralité de doses de stimulation électrique avec un premier ensemble de paramètres de stimulation, le procédé comprenant en outre :
la détermination, en fonction du fait que la première pluralité de doses de stimulation électrique a évoqué des potentiels neuronaux chez le patient, d'un second ensemble de paramètres de stimulation.

4. Support de stockage lisible par ordinateur selon la revendication 3, dans lequel la détermination du second ensemble de paramètres de stimulation comprend :
en réponse à la détermination que la première pluralité de doses n'a pas évoqué de potentiels neuronaux chez le patient, la détermination du second ensemble de paramètres de stimulation pour comporter une amplitude supérieure à une amplitude du premier ensemble de paramètres de stimulation.

5. Support de stockage lisible par ordinateur selon la revendication 3, dans lequel le procédé comprend en outre :
l'administration, par le dispositif de stimulation implantable et avec le second ensemble de paramètres de stimulation, d'une seconde pluralité de doses de stimulation électrique.

6. Support de stockage lisible par ordinateur selon l'une quelconque des revendications 1 ou 2, dans lequel l'administration de la pluralité de doses de stimulation électrique comprend l'administration, à un premier moment, d'une première pluralité de doses de stimulation électrique avec un premier ensemble de paramètres de stimulation, le procédé comprenant en outre :
la détermination, en fonction du fait que la première pluralité de doses de stimulation électrique a évoqué des potentiels neuronaux chez le patient, d'un état pathologique du patient.

7. Support de stockage lisible par ordinateur selon l'une quelconque des revendications précédentes, dans lequel l'administration des doses de stimulation électrique comprend :
l'administration des doses de stimulation électrique à une fréquence supérieure ou égale à 100 Hz.

8. Système comprenant :
une mémoire (211) ; et
un circuit de traitement (210) configuré pour :
amener un dispositif de stimulation implantable à administrer une pluralité de doses de stimulation électrique à un patient ;
recevoir une pluralité de signaux électriques, dans lequel la pluralité de signaux électriques comprend un signal électrique respectif pour chaque dose respective de la pluralité de doses ; et
la détermination d'une variation dans le temps en fonction de la pluralité de signaux électriques ;
déterminer, en fonction de l'application d'une quantité seuil à la variation de la pluralité de signaux électriques, si la pluralité de doses de potentiels neuronaux évoqués par stimulation électrique indiquent une réponse du patient à la pluralité de doses de stimulation électrique.

9. Système selon la revendication 8, dans lequel le circuit de traitement (210) est en outre configuré pour soit :
déterminer la variation en tant qu'écart type de la pluralité de signaux électriques ; ou
déterminer la variation en tant que variance de la pluralité de signaux électriques.

10. Système selon l'une quelconque des revendications 8 ou 9, dans lequel, pour amener le dispositif de stimulation implantable à administrer la pluralité de doses de stimulation électrique, le circuit de traitement est configuré pour amener le dispositif de stimulation implantable à administrer une première pluralité de doses de stimulation électrique avec un premier ensemble de paramètres de stimulation, et dans lequel le circuit de traitement est en outre configuré pour :
déterminer, en fonction du fait que la première pluralité de doses de stimulation électrique a évoqué des potentiels neuronaux chez le patient, un second ensemble de paramètres de stimulation.

11. Système selon la revendication 10, dans lequel, pour déterminer le second ensemble de paramètres de stimulation, le circuit de traitement (210) est en outre configuré pour :
déterminer, en réponse à la détermination que la première pluralité de doses n'a pas évoqué de potentiels neuronaux chez le patient, le second ensemble de paramètres de stimulation pour comporter une amplitude supérieure à une amplitude du premier ensemble de paramètres de stimulation.

12. Système selon la revendication 10, dans lequel le circuit de traitement (210) est en outre configuré pour :
amener le dispositif de stimulation implantable à administrer, avec le second ensemble de paramètres de stimulation, une seconde pluralité de doses de stimulation électrique.

13. Système selon l'une quelconque des revendications 8 ou 9, dans lequel, pour amener le dispositif de stimulation implantable à administrer la pluralité de doses de stimulation électrique, le circuit de traitement (210) est configuré pour amener le dispositif de stimulation implantable à administrer, à un premier moment, une première pluralité de doses de stimulation électrique avec un premier ensemble de paramètres de stimulation, et dans lequel le circuit de traitement (210) est en outre configuré pour :
déterminer, en fonction du fait que la première pluralité de doses de stimulation électrique a évoqué des potentiels neuronaux chez le patient, un état pathologique du patient.

14. Système selon la revendication 13, dans lequel le circuit de traitement (210) est en outre configuré pour :
amener le dispositif de stimulation implantable à administrer, à un second moment qui est après le premier moment, une seconde pluralité de doses de stimulation électrique avec le premier ensemble de paramètres de stimulation ; et
déterminer, en fonction du fait que la seconde pluralité de doses de stimulation électrique a évoqué des potentiels neuronaux chez le patient, si l'état pathologique du patient a changé.

15. Système selon la revendication 8, dans lequel le circuit de traitement (210) est en outre configuré pour :
ajuster, en fonction du fait que la pluralité de doses de stimulation électrique a évoqué des potentiels neuronaux chez le patient, l'administration ultérieure de la stimulation électrique à un patient.
